# EUROPEAN PATENT APPLICATION

(11) **EP 3 335 664 A1**
(43) Date of publication of application: **20.06.2018**
(21) Application number: 16204406.9
(22) Date of filing: 15.12.2016
(51) Int. Cl.: A61B 90/00, A61B 34/20

(54) **FIDUCIAL MARKER AND METHOD OF MANUFACTURING A FIDUCIAL MARKER**

(71) Applicant: Carl Zeiss Industrielle Messtechnik GmbH, 73447 Oberkochen (DE)
(72) Inventor: SMIGIELSKI, Arkadiusz, 76-251 Kobylnica (PL); BODJANSKI, Mariusz, 05-220 Zielonka (PL); KRASZEWSKI, Maciej, 76-200 Slupsk (PL)
(74) Representative: Patentanwälte Bressel und Partner mbB

(57) **Abstract**

The invention relates to a fiducial marker (1; 21), in particular for determination of position and/or orientation in medical imaging systems and/or in machine arrangements comprising movable parts, wherein the fiducial marker (1; 21) comprises a pattern (2), the position and/or orientation of which is to be determined. The pattern (2) is transparent and the fiducial marker (1; 21) comprises a radiation source (3; 23) connected directly and/or indirectly with the pattern (2). During operation, radiation emitted by the radiation source (3; 23) is transmitted through the pattern (2) from a back side to a front side of the pattern (2).

## Description

The invention relates to a fiducial marker and to a method of manufacturing a fiducial marker. In particular, the marker can be used for the determination of its position and/or its orientation, especially as part of a tracking system for tracking movement of a movable part. Markers are used especially in medical imaging systems and/or in machine arrangements comprising movable parts. The fiducial markers according to the present invention comprise a pattern, the position and/or orientation of which is to be determined.

Therefore, when the marker is attached to an object (e.g. to a movable part of a machine or of a machine arrangement or to a human or animal patient to be treated and/or to be examined), the object's position and/or orientation can be determined. Often, a plurality of markers is attached and/or fixed to the object. Typically, the marker is observed by at least one digital camera, which means that at least one image of the marker is produced by the camera and the at least one image is evaluated. By this evaluation, which may be performed by digital data processing using information about the position and orientation of the camera, the position and/or orientation of the at least one marker is determined. In particular, the tracking of the position of at least one marker using at least one digital camera is known in order to measure the position of an object in a three-dimensional (3D) space.

Two types of markers may be distinguished: passive markers that reflect incident radiation from a separate radiation source, and active markers that produce and emit radiation on their own. The marker shall be reliably detectable, in particular in the at least one image generated by the at least one camera. This shall apply regardless of the position and orientation of the marker as long as the marker is visible to the respective camera. Especially when the object to which the marker is attached can move within a large 3D-volume, this requirement is difficult to fulfill.

Passive markers need to be well illuminated in order to be detectable. This requires bright, uniform illumination of the entire 3D-Volume, which means considerable effort and demanding technical equipment. Even in this case, the radiation intensity of the radiation which impinges on the detector (in particular camera) decreases which increasing distance to the marker and with increasing angle of the line of view relative to the direction perpendicular to the marker's surface, in case of two-dimensional (2D) markers.

Active markers do not require external illumination. Typically, they consist of a light source which may comprise a single or a plurality of light generating elements, such as light emitting diodes. Compared to more elaborate passive markers, the appearance of active markers is simple. Therefore, passive markers have been used when complex patterns are required by the position and/or orientation determining system in order to increase robustness and accuracy of determination results, especially of object tracking.

It is an object of the present invention to provide a fiducial marker and a method of manufacturing a fiducial marker that allow for robust and accurate determination of marker position and/or orientation without the need for a bright, uniform illumination within the entire 3D-volume in which the marker may be positioned.

According to a basic idea of the present invention, a fiducial marker comprises a radiation source that is connected directly and/or indirectly with a pattern, the position and/or orientation of which is to be determined. In case of an indirect connection, the connecting part or the connecting parts is/are also part(s) of the fiducial marker, i.e. even in case of an indirect connection, the radiation source and the pattern are parts of a single-piece unit (the fiducial marker) that can be fixed to an object. Compared to uniform markers, a pattern comprises more structure information and is in particular suitable for unambiguously determining not only the position but also the orientation of the marker from a single image. In addition, the velocity of a movement of the marker relative to a camera or arrangement of cameras can be determined from a single image or from a set of images taken at the same time.

Since the marker has its own radiation source, a bright and uniform illumination of the complete 3D-volume where the maker may be positioned is not required. Therefore, the marker can be used in large 3D-volumes, e.g. rooms, such as warehouses, production facilities. Furthermore, the marker can be used in environments of the marker where a sufficient irradiation of the 3D volume using separate radiation sources requires too much effort, because a significant percentage of the radiation is absorbed and/or scattered in the environment, such as by water or other liquids.

In addition, the radiation source and thereby the radiation which is produced by the radiation source can be adapted especially to the pattern and to the requirements of determining the marker(s) position and/or orientation. This adaptation is not limited to the mechanical construction and to the arrangement of the radiation source relative to the pattern, but optionally includes the adaption of the radiation intensity, i.e. the intensity may vary depending on time and/or the arrangement of the marker relative to the position and/or orientation determining system.

Furthermore, the pattern of the fiducial marker is transparent and/or reflective so that the radiation produced by the radiation source illuminates the pattern from its back side and/or its front side so that an image of the pattern is detectable by any device or arrangement of devices on the front side. In particular, the pattern may comprise a surface detectable from the front side wherein this front side surface extends in a plane or along a plane. In particular, the whole front side surface extends in the plane or along the plane, i.e. the front side surface does not extend along a curve.

The radiation source of the fiducial marker may comprise a single radiation generating element, such as an LED (light emitting diode). The term "light" in this context is not restricted to visible light. Generally speaking, the radiation generating element of the radiation source may produce electromagnetic radiation in any range of wavelength. In practice, when using at least one camera for generating at least one image of the marker, radiation having a wavelength in the visible and/or in the infrared range is preferred. In any case, it is preferred that the radiation source of the fiducial marker comprises a plurality of radiation generating elements. In particular, at least one group of radiation generating elements can be directly connected to each other. In case of more than one group, the radiation source can be an arrangement of these groups, optionally with a distance between the groups. For example, one group of radiation generating elements can be arranged so as to extend along a first section of the marker's outline and a second group can be arranged so as to extend along a second section of the marker's outline, wherein the first section and the second section are straight sections extending at an angle to each other, for example at an angle of at least 30 degrees, in particular at least 60 degrees and especially at an angle of 90 degrees to each other. According to another embodiment, a single group of radiation generating elements or a plurality of groups may be arranged - if viewed from the front side of the pattern - behind the pattern, i.e. on the back side of the pattern.

In particular, a nearly uniform radiation intensity distribution of the radiation produced by the radiation source that impinges on the pattern, or that impinges on a carrier of the pattern, can be produced. Especially, the intensity distribution can be made even more uniform by using at least one diffusing body or layer through which the generated radiation is transmitted before it impinges on the back side surface or front side surface or pattern. The carrier may comprise a diffusing layer made of a material that scatters radiation entering the diffusing layer, wherein the diffusing layer is arranged on the back side of the pattern. In particular, the diffusing layer may form the surface of the carrier on which the pattern is fixed. Alternatively, the diffusing layer may be arranged on a back side of the carrier, in particular a back side of the layer where the radiation emitted by the radiation source enters the carrier. However, it is also possible that there is a plurality of diffusing layers, for example one diffusing layer next to the pattern and one diffusing layer on the back side of the carrier.

In particular, the following is proposed: A fiducial marker, in particular for determination of position and/or orientation in medical imaging systems and/or in machine arrangements comprising movable parts, wherein the fiducial marker comprises a pattern, the position and/or orientation of which is to be determined. The pattern is transparent and the fiducial marker comprises a radiation source connected directly and/or indirectly with the pattern. During operation of the fiducial marker, radiation emitted by the radiation source is transmitted through the pattern from a back side to a front side of the pattern.

In addition or alternatively to the transparent pattern, the pattern may be reflective. In this case, the radiation source, which is connected directly and/or indirectly with the pattern, emits radiation that is irradiated onto the pattern and is reflected by the pattern during operation.

In particular, the pattern comprises a first area having a first transmittance and a second area having a second transmittance wherein the first and the second transmittance differ. In case of a reflective pattern, there is a first area having a first reflectance and a second area having a second reflectance, wherein the first and the second reflectance differ. For example, the first transmittance and the second transmittance, respectively the first reflectance and the second reflectance, differ by at least 0.2 (i.e. 20 %), in particular by at least 0.4 and preferably at least 0.6.

Consequently, any device (such as a digital camera) that observes the pattern in order to determine the position and/or orientation of the pattern, can observe the different areas and in the result of the observation (such as the digital image) the first and second area are distinguishable using the different effects of the transmittances/reflectances. In particular, a spatial position and an orientation of the pattern in three-dimensional space (3D) are determinable. For example, in a digital image produced by a camera, the first area may be represented by first pixels having a first image value (such as a greyscale or color value) and the second area may be represented by second pixels having a second image value that differs from the first image value. Alternatively, the first pixels may having a first image value

The pattern may have any shape suitable for the determination of position and/or orientation. In particular, the pattern may be a two-dimensional pattern. E.g., the shape may be circular, rectangular, triangular, cross-shaped. However, it is not excluded that the pattern is a one-dimensional pattern. Furthermore, it is possible that the pattern is a three-dimensional pattern. The dimension is related to the information that can be observed of a device such as a camera.

The transparent and/or reflective pattern may consist of a thin structure, which means, that the height of the pattern is smaller than one tenth (in particular smaller than one hundredth) of the width and/or the length of the pattern.

The different areas of the pattern having different optical properties (transmittance and/or reflectance) can be arranged in various ways, e.g. so that zones of constant or similar optical properties extend in circumferential or radial direction when considering a circular pattern. The transmittance and/or reflectance may refer to a specific, predetermined radiation wavelength, a range of wavelengths and/or plurality For example in case of the first and second areas mentioned above, there may be several first and several second areas, wherein the first and second areas form a repeating structure, i.e. there is at least one first area in between two second areas and vice versa, e.g. repeating structure regarding shape and/or size, e.g. repeating areas in circumferential direction.

The different areas within the pattern can be distinct by continuous transitions or by discrete boundaries. For example, the transitions between inner parts of the pattern may be continuous, whereas the transition between an inner area and an outer area of the same pattern may be discrete.

One example of a pattern that can be used as a transparent pattern and/or as a reflective pattern in a fiducial marker according to the present invention, is described by WO 2016/071227 A1: The marker is an optical-tracking marker to be attached to an object. The marker an inner marker body having an elliptical shape and a geometric center, wherein the inner marker body is filled with color spectrum points radially spread with respect to the geometric center. A color value of each spectrum point of the inner marker body is calculated as a function of an angle defined between a horizontal line crossing the central point and a further line crossing the central point as well as the respective spectrum point.

In case of a transparent pattern, the pattern may comprise opaque or nearly opaque areas corresponding to a radiation transmittance of zero or almost zero. In case of a reflective pattern, the pattern may comprise non-reflective or nearly non-reflective areas corresponding to a reflectance of zero or almost zero. Both cases particularly refer to an outer area of a circular or polygonal pattern.

In particular, the pattern may be fixed on a surface of a carrier that is located on the back side of the pattern, wherein the carrier is made of transparent material and wherein, during operation, the radiation emitted by the radiation source is transmitted through the transparent material of the carrier. For example, the carrier may be a plate-shaped body. The carrier may be held in position by and/or within a frame and/or housing of the fiducial marker. The carrier may help to stabilize the form of the pattern while still allowing radiation to pass through the transparent pattern.

The pattern may be a pre-fabricated pattern that is attached or fixed during manufacture of the fiducial marker to at least one other part of the marker, in particular to the carrier, as mentioned before. Alternatively, the pattern can be produced by printing the pattern directly onto a carrier. In particular, it is possible that the radiation-transparent carrier, or in case of the pattern being a reflective pattern optionally an opaque carrier, is provided first and the pattern is then printed or otherwise generated onto the surface of the carrier. In particular, treating the surface of the carrier by appropriate means (such as etching or treating with chemicals in order to modify the optical properties) is possible. In addition or alternatively, at least a part of the pattern (e.g. a prefabricated pattern) may be integrated in the carrier, i.e. may be embedded in transparent material of the carrier, so that the pattern is completely or partly enclosed by material of the carrier.

In many cases, it is desired that the pattern can be observed from different viewing directions on the front side of the pattern. Therefore, it is advantageous in some cases that the radiation intensity on the front side of the pattern as an approximately uniform intensity distribution within at least major parts of the hemisphere. When the radiation source emits radiation onto the pattern's back side, it is therefore of advantage to scatter the radiation, which means, that the intensity distribution is modified to become more uniform. One option is to use a diffusing layer or diffusing body made of a material that scatters radiation which enters or has entered the diffusing layer or body. Such a diffusing layer or body can be arranged on the back side of the pattern in case of a transparent pattern. In particular, such a diffusing layer may be a surface layer covering the surface of the carrier mentioned above. Preferably, the surface layer is a layer of the carrier to which the pattern is fixed.

A scattering layer can be realized as a separate component consisting of a transparent material and comprising particles causing scattering of radiation. These particles may have a refractive index significantly different from the surrounding matrix. An example is milk glass, which may contain tricalcium phosphate or/and fluoride or/and tin oxide. The same mechanism also applies to milky acrylic glass by using dye, for instance.

In addition or alternatively a scattering effect can also be realized by a roughened surface, e.g. the surface on the back side and/or the front side of the carrier, adapted to scatter the radiation which is transmitted through the transparent material of the carrier and enters the pattern. For example, radiation is scattered by small planar segments of the roughened surface pointing in various directions.

The diffusing layer may be a layer having a constant thickness. In addition or alternatively, the thickness of the transparent carrier may be constant. The direction in which the thickness is to be measured is the direction perpendicular to the surface to the pattern on the back side of the pattern.

In case of the pattern being fixed to the surface of the transparent carrier, the carrier and the pattern may form a common surface oriented to the front side of the pattern, wherein, during operation, radiation emitted by the radiation source is also transmitted through the carrier to the front side of the pattern without passing the pattern. Therefore, an image taken of the marker (e.g. recorded by a camera) may show areas of the marker corresponding to the carrier sideways of the pattern and as well may show the pattern. In some cases, this may help to provide high contrast in the image or appearance of the marker at the outline of the pattern. This in turn may help to unambiguously determine the outline of the pattern. The contrast is particularly high, if the outline of the pattern is defined by an area of the pattern which has a low transmittance and/or reflectance, i.e. appears dark in the image. Preferably, this area that defines the outline has a transmittance and/or reflectance of maximal 0.03, preferably maximal 0.015 and most preferred maximal 0.01.

Especially in the case mentioned before, but also in other cases, the pattern may comprise a peripheral area forming a peripheral surface of the pattern on the front side and having a constant (not position-dependent) transmittance, wherein the peripheral area encloses an inner area of the pattern forming an inner part of the surface of a pattern on the front side and wherein the peripheral part of the surface completely encloses the inner part of the surface of the pattern on the front side. Again, this may help to identify the outline of the pattern.

The radiation source may be arranged, if viewed from the front side, behind the pattern. In addition or alternatively, at least a part of the radiation source may be arranged, if viewed from the front side, sideways of the pattern. In both cases, transparent material, such as of the carrier and/or diffusing layer or body mentioned above may be placed along the path of radiation in between the respective part of the radiation source and the back side of the pattern. In particular, the marker may comprise a body arranged on the back side of the pattern, wherein, during operation, radiation emitted by the radiation source enters the body, is reflected on at least one part of the surface of the body and is then transmitted to the back side of the pattern and through the pattern to the front side of the pattern. The at least one part of the surface of the body may comprise a reflective coating or may be combined with a reflective body so that the radiation is reflected and is thereby kept within the body and/or re-enters the body. However, at least the part of the surface of the body through which the radiation leaves the body in order to enter the pattern is not provided with a reflective coating or combined with a reflective body.

Some of the embodiments of the fiducial marker described before and in the following are embodiments that require the pattern to be transparent. At least some of these embodiments and other embodiments described above and below may comprise a reflective pattern. In particular, a reflective pattern may be fixed on a surface of a carrier (in particular an opaque carrier), thereby covering only a first part of the carrier, wherein another, second part of the carrier that is not covered by the pattern is reflective (for example white), wherein the second part of the carrier and the pattern form a common reflective surface on a front side of the pattern and wherein, during operation, radiation emitted by the radiation source is also reflected by the second part of the carrier. For example, this embodiment may comprise also at least one of the features described above in connection with high contrast of the outline of the pattern. In particular, the peripheral area of the pattern mentioned above may have a constant reflectance instead of a constant transmittance as mentioned above.

Furthermore, a method of manufacturing a fiducial marker is proposed, in particular a fiducial marker for determination of position and/or orientation in medical imaging systems and/or in machine arrangements comprising movable parts, wherein the method comprises providing a transparent and/or reflective pattern, the position and/or orientation of which is to be determined. Furthermore, the method comprises providing a radiation source and connecting the fiducial marker directly and/or indirectly with the pattern.

Embodiments of the manufacturing method follow from the description of the fiducial marker. In particular, the pattern may be fixed on a surface of a transparent carrier wherein the pattern is fixed on a surface of a transparent carrier on a back side of the pattern, wherein the radiation source is arranged in such a manner that, during operation, radiation emitted by the radiation source is transmitted through the transparent material of the carrier and is then transmitted through the pattern from the back side to a front side of the pattern.

In addition or alternatively, the pattern may be printed on the surface of the radiation-transparent carrier.

Examples of the invention will be described with reference to the attached figures:
- Fig. 1: shows an isometric view of a first example of a fiducial marker having a transparent pattern,
- Fig. 2: shows an isometric view of a second example of a fiducial marker having a transparent pattern,
- Fig. 3: shows a cross-section through the transparent pattern and its carrier of Fig. 2 for a specific embodiment of the carrier,
- Fig. 4: shows an isometric view of a fiducial marker having a reflective pattern,
- Fig. 5: shows an example of an arrangement comprising a machine with movable parts and cameras for tracking movement of one of the movable parts of the machine.

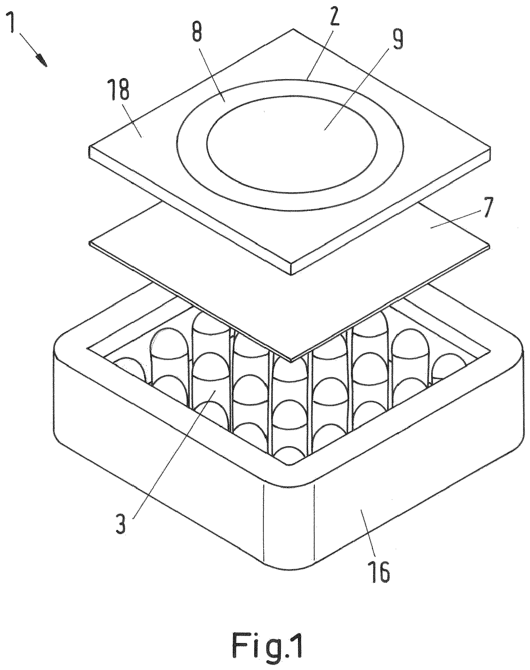

Fig. 1 shows an exploded view of a fiducial marker 1 that comprises a housing 16 that includes an array of light emitting diodes as a radiation source 3. The housing 16 has a rectangular configuration with rounded corners that defines four outer side surfaces. Means for attaching the bottom surface of the housing 16 to any object may be present, but are not visible in Fig. 1. The upper surface of the radiation source 3 is covered by a radiation diffusing layer 7, on top of which a carrier 18 is placed that carries a transparent pattern 2.

In particular, the upper surface of the radiation source 3 may be a surface extending within a plane and the lower surface of the diffusing layer contacts the upper surface of the radiations source 3 without significant voids in between the contacting surfaces.

The diffusing layer 7 may be an integral part of the carrier 18 or may be a separate diffusing layer. In case of being a separate diffusing layer, the upper surface of the diffusing layer 7 and the lower surface of the carrier 18 also contact each other over the whole cross-section without significant voids.

The material of the carrier 18 and the material of the diffusing layer 7 are transparent materials so that the radiation emitted by the radiation source 3 is transmitted through the diffusing layer 7 and through the carrier 18 in upward direction towards the upper side of the fiducial marker 1 which is the "front side" of the pattern 2. However, most of the radiation that is emitted by the radiation source 3 and that is transmitted through the carrier 18 first impinges on the back side surface of the pattern 2 and is transmitted through the pattern 2. Depending on the transmittance of the respective area of the pattern 2, the radiation intensity is decreased and a correspondingly lower radiation intensity can be observed on the front side. Since the transmittance of the pattern depends on the respective area, the transmittance structure of the pattern can be observed, e.g. by a camera. Another portion of the radiation that is transmitted through the carrier 18 passes through the peripheral area of the carrier 18, i.e. does not pass through the pattern 2. Therefore, the radiation distribution on the front side of the pattern 2 provides information on the transmittance of the pattern 2 corresponding to the structure of the pattern 2 and, in addition, since the peripheral area of the carrier 18 is also transparent, also provides information on the outline of the pattern 2. Therefore, not only the pattern structure, but also the outline can be evaluated using any image or plurality of images taken of the fiducial marker from the front side.

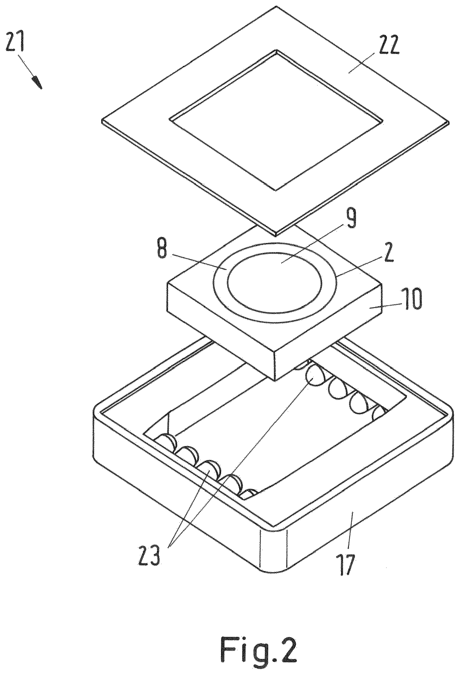

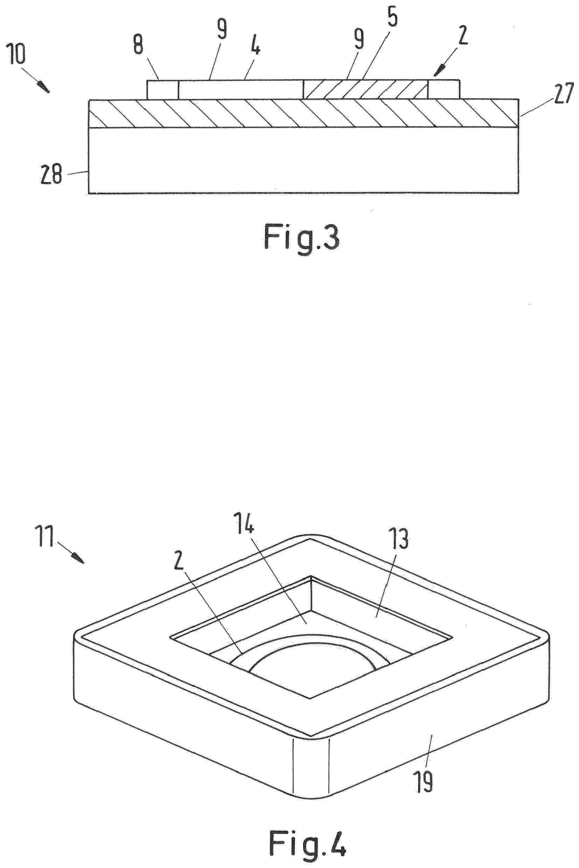

In the specific embodiment shown in Fig. 1, the pattern 2 has a circular shape with a circular ring 8 defining the outline of the pattern 2 and with an inner circle 9 comprising a complex pattern structure that comprises more structure information compared to uniform markers and is in particular suitable for unambiguously determining not only the position but also the orientation of the marker from a single image. While the transmittance of the circular ring 8 is constant and is preferably smaller then 0.02, the transmittance of the inner circle 9 varies with the location within the inner circle 9. However, the specific example shown in Fig. 1 and as well in Fig. 2 and Fig. 4, does not limit the invention, but the fiducial markers described in connection with Fig. 1 to 4 may comprise another pattern instead of the pattern shown in the figures. Alternatively, embodiments of the fiducial marker may comprise more than one pattern, for example a plurality of patterns fixed to the upper surface of the carrier shown in Fig. 1, Fig. 2 or Fig. 4. These patterns may be placed at a distance to each other which means that the area in between the patterns has the optical properties (transmittance and/or reflectance) of the carrier only.

Fig. 2 shows an exploded view of a fiducial marker 21 that also comprises, like the fiducial marker 1 of Fig. 1, a transparent pattern 2. The fiducial marker 21 comprises a housing 17 having a rectangular cross-section with rounded corners, but in contrast to the fiducial marker 1 in Fig. 1 a radiation source 23 of the fiducial marker 21 comprises two groups of LEDs on opposite side margins of the housing 17. There are reflective inner side surfaces, in particular white surfaces on opposite sides of the housing 17, extending from the side of a first of the groups of LEDs to the opposite where the second group of the LEDs is located. When the fiducial marker 21 is readily mounted, a carrier 10 in the shape of a cuboid is arranged in between the opposite groups of LEDs. The material of the carrier 10 is transparent. Preferably, the inner bottom surface of the housing 17 that faces upwards also has reflective properties (e. g. is white). Therefore, during operation, the radiation generated by the radiation source 23 is partly reflected by the reflective inner side surfaces and is preferably also partly reflected by the inner bottom surface so that a essentially uniform radiation intensity distribution is generated and the corresponding radiation is transmitted upwards through the carrier 10. Similarly to the fiducial marker 1 shown in Fig. 1, some of the radiation that is transmitted through the carrier also passes through the pattern 2 from its back side to its front side and some of the radiation passes through the peripheral part of the carrier without passing through the pattern 2. Therefore, the appearance of the pattern 2 and of the peripheral area of the carrier next to the outline of the pattern 2 is the same or very similar as for the appearance of the pattern 2 and peripheral area in Fig. 1.

The fiducial marker 21 of Fig. 2 may also comprise a cover 22 which covers the side margins of the housing 17 that comprise the opposite groups of LEDs and the areas with the reflective inner side surfaces, but comprises a rectangular central cut-out leaving the surface of the pattern 2 and of the peripheral area of the carrier 10 open to the upper side of Fig. 2 (the front side of the pattern).

Although not preferred, at least one of the fiducial markers shown in Fig. 1, Fig. 2 and Fig. 4 may comprise an additional transparent cover without radiation-diffusing properties so that the pattern can be protected against damage and dirt.

The carrier 10 of the fiducial marker 21 shown in Fig. 2 may comprise means for scattering radiation and/or may be combined with an additional layer or body that scatters radiation, in order to produce an even more uniform radiation intensity distribution. One example is shown in Fig. 3. In this example, the carrier 10 comprises a lower part 28 and an upper part 27. While there is no significant scattering of radiation (i.e. no diffusing property) in the lower part 28, the upper part 27 scatters radiations and therefore produces the more uniform radiation intensity distribution.

Fig. 3 also schematically shows the pattern 2 of Fig. 2 with the circular ring 8 and the inner circle 9. In the specific cross-section of Fig. 3, the inner circle 9 comprises a first area 4 and a second area 5 having different transmittances.

Fig. 4 shows a fiducial marker 11 having a housing 19, which is similar to the housing 17 of the fiducial marker 21 of Fig. 2. The radiation source 13 also comprises a plurality of groups of LEDs in side margins of the housing. However, in contrast to Fig. 2, the housing 19 of Fig. 4 comprises four side margins of equal size, one side margin along each of the four straight sections of the housing which define the outline of the housing 19. There are preferably groups of LEDs in at least two of the four side margins. However, the inner side surface of at least one of the margins can be reflective.

The pattern 2 of the fiducial marker 11 is placed at the inner bottom surface of the housing 19 so that the radiation generated by the radiation source 13 impinges from the front side of the pattern onto the pattern and onto a peripheral surface area of the inner bottom surface so that the radiation is reflected to the front side and a corresponding image can be taken.

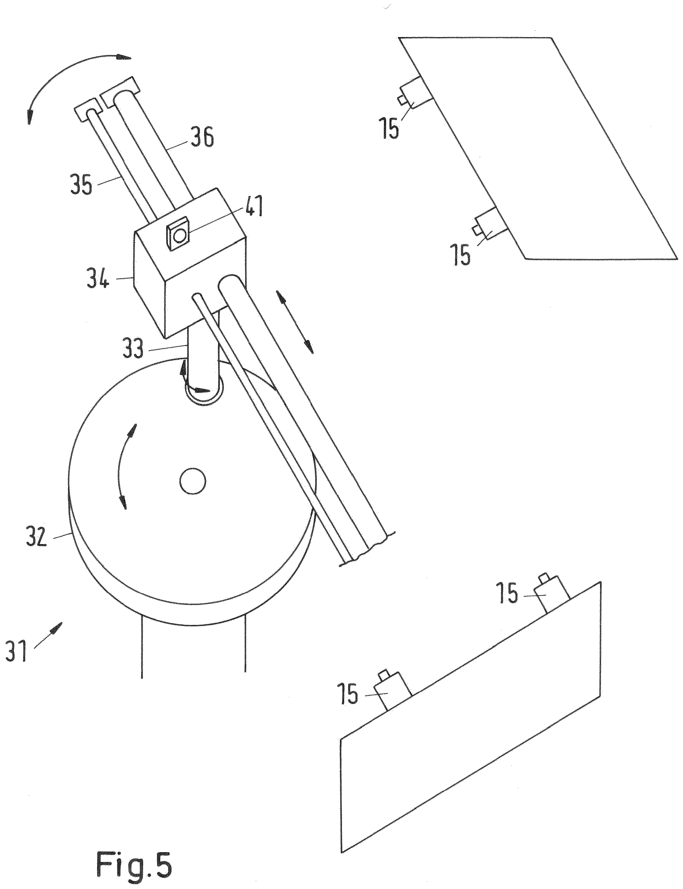

Fig. 5 shows an example of a machine 31 that comprises several moving parts. In addition, the arrangement shown in Fig. 5 comprises four cameras 15, wherein in each case two cameras 15 are arranged as a set of stereo cameras that can take two images at the same time from different angles of views of the same object. In other words, the same object is captured by the images viewed from different points of view.

The machine comprises a first movable part, a turn table 32 that can rotate about a first axis of rotation which extends in vertical direction of Fig. 5. In the peripheral area of the turn table 32, there is a shaft 33 extending with its longitudinal axis parallel to the first axis of rotation. The shaft 33 can be rotated about a second axis of rotation which is the central longitudinal axis of the shaft 33. At the top of the shaft 33, there is a movable block 34. This movable block 34 is moved according to the rotation performed by the turn table 32, but its movement is confined by two rods 35, 36 that extend in straight direction through the movable block 34. The movable block 34 can move relative to the rods 35, 36, as indicated by a straight double-arrow, along the longitudinal axes of the rods 35, 36. The ends of the rods 35, 36 at the bottom part of Fig. 5, which ends are not shown in Fig. 5 are connected to a joint that is fixed relative to the cameras 15, so that the movable block 34 not only performs a straight shift along the longitudinal axes of the rods 35, 36, but also performs a pendular movement as indicated by a bent double-arrow at the top of Fig. 5 when the turn table 32 rotates about its first axis of rotation.

A fiducial marker 41, such as one of the fiducial markers 1, 11, 21 shown in Fig. 1, Fig. 2 and Fig. 4, is fixed to the upper surface of the movable block 34. The front side of the fiducial marker 41 is visible to each pair of cameras 15 and the cameras 15 continuously generate digital images of the front side of the marker 41. These images may be transferred to an evaluation device (not shown in Fig. 5) of a tracking system for determining the position and orientation of the marker 41 and thereby of the movable block 34 as a function of time.

## Claims

1. A fiducial marker (1; 21), in particular for determination of position and/or orientation in medical imaging systems and/or in machine arrangements comprising movable parts, wherein the fiducial marker (1; 21) comprises a pattern (2), the position and/or orientation of which is to be determined,
**characterized in that**
- the pattern (2) is transparent,
- the fiducial marker (1; 21) comprises a radiation source (3; 23) connected directly and/or indirectly with the pattern (2),
- during operation, radiation emitted by the radiation source (3; 23) is transmitted through the pattern (2) from a back side to a front side of the pattern (2).

2. The fiducial marker (1; 21) of claim 1, wherein the pattern (2) comprises a first area (4) having a first transmittance and a second area (5) having a second transmittance, wherein the first and the second transmittance differ.

3. The fiducial marker (1; 21) of claim 1 or 2, wherein the pattern (2) is fixed on a surface of a carrier (10; 18) on the back side of the pattern, wherein the carrier (10; 18) is made of transparent material and wherein, during operation, the radiation emitted by the radiation source (3; 23) is transmitted through the transparent material of the carrier.

4. The fiducial marker (1; 21) of claim 3, wherein the surface of the carrier (10; 18) comprises a rough surface structure adapted to scatter the radiation which is transmitted through the transparent material and enters the pattern (2).

5. The fiducial marker (1; 21) of claim 3 or 4, wherein the carrier (10; 18) comprises a diffusing layer (7; 27) made of a material that scatters radiation entering the diffusing layer (7; 27), wherein the diffusing layer (7; 27) is arranged on the back side of the pattern (2).

6. The fiducial marker (1; 21) of one of claims 3 to 5, wherein the carrier (10; 18) and the pattern (2) form a common surface oriented to the front side of the pattern (2) and wherein, during operation, radiation emitted by the radiation source (3) is also transmitted through the carrier (10; 18) to the front side of the pattern (2) without passing the pattern (2).

7. The fiducial marker (1; 21) of one of claims 1 to 6, wherein the pattern (2) comprises a peripheral area (8) forming a peripheral part of the surface of the pattern (2) on the front side and having a constant transmittance, wherein the peripheral area (8) encloses an inner area of the pattern forming an inner part (9) of the surface of the pattern (2) on the front side and wherein the peripheral part (8) of the surface completely encloses the inner part (9) of the surface of the pattern (2) on the front side.

8. The fiducial marker (1; 21) of one of claims 1 to 7, wherein the radiation source (3, 23) is arranged, if viewed from the front side, behind the pattern (2).

9. The fiducial marker (1; 21) of one of claims 1 to 8, wherein the radiation source (23) is arranged, if viewed from the front side, sideways of the pattern (2).

10. The fiducial marker of claim 9, further comprising a body (10; 18) arranged on the back side of the pattern (2), wherein, during operation, radiation emitted by the radiation source (3; 23) enters the body (10; 18), is reflected on at least one surface of the body (10; 18) and is then transmitted through the pattern (2) from the back side to the front side of the pattern (2).

11. A fiducial marker (11), in particular for determination of position and/or orientation in medical imaging systems and/or in machine arrangements comprising movable parts, wherein the fiducial marker (11) comprises a pattern (2), the position and/or orientation of which is to be determined,
**characterized in that**
- the pattern (2) is reflective,
- the fiducial marker (11) comprises a radiation source (13) connected directly and/or indirectly with the pattern (2),
- during operation, radiation emitted by the radiation source (13) is irradiated onto the pattern (2) and is reflected by the pattern (2).

12. The fiducial marker (11) of claim 11, wherein the pattern (2) is fixed on a surface of a carrier (14), thereby covering only a first part of the carrier (14), wherein a second part of the carrier (14) that is not covered by the pattern (2) is reflective, wherein the second part of the carrier (14) and the pattern (2) form a common reflective surface on a front side of the pattern (2) and wherein, during operation, radiation emitted by the radiation source (13) is also reflected by the second part of the carrier (14).

13. A method of manufacturing a fiducial marker, in particular a fiducial marker for determination of position and/or orientation in medical imaging systems and/or in machine arrangements comprising movable parts, comprising:
- providing a pattern (2), the position and/or orientation of which is to be determined,
**characterized by**
- providing the pattern (2) as a transparent and/or reflective pattern,
- providing a radiation source (3; 13; 23),
- connecting the fiducial marker (1; 11; 21) directly and/or indirectly with the pattern.

14. The method of claim 13, wherein the transparent pattern (2) is fixed on a surface of a transparent carrier (10; 18) on a back side of the pattern (2) and wherein the radiation source (3; 23) is arranged in such a manner that, during operation, radiation emitted by the radiation source (3; 23) is transmitted through the transparent material of the carrier (10; 18) and is then transmitted through the pattern (2) from the back side to a front side of the pattern (2).

15. The method of claim 14, wherein the pattern (2) is printed on the surface of the transparent carrier (10; 18).
